# EUROPEAN PATENT APPLICATION

(11) **EP 4 516 293 A1**
(43) Date of publication of application: **05.03.2025**
(21) Application number: 23795500.0
(22) Date of filing: 26.04.2023
(51) Int. Cl.: A61K 9/22, A61K 31/5377, A61K 47/38, A61K 47/36, A61K 47/04, A61K 47/10, A61P 1/16, A61P 31/14, A61P 31/20

(54) **DRUG SUSTAINED-RELEASE TABLET FOR TREATING LIVER DISEASE, PREPARATION METHOD THEREFOR AND USE THEREOF**

(30) Priority: 28.04.2022 CN 202210459865
(71) Applicant: Changchun Intellicrown Pharmaceutical Co., Ltd., Jilin 130507 (CN); INSTITUTE OF MATERIA MEDICA, CHINESE ACADEMY OF MEDICAL SCIENCES, Beijing 100050 (CN)
(72) Inventor: WU, Song, Beijing 100050 (CN); ZHANG, Chi, Beijing 100050 (CN); LIU, Bo, Beijing 100050 (CN); YANG, Qingyun, Beijing 100050 (CN); ZHANG, Wenxuan, Beijing 100050 (CN); HAN, Zunsheng, Beijing 100050 (CN); FENG, Jing, Beijing 100050 (CN); ZHANG, Jie, Beijing 100050 (CN); ZHANG, Kun, Beijing 100050 (CN); LI, Tianlei, Beijing 100050 (CN); XIA, Jie, Beijing 100050 (CN); SUN, Hua, Beijing 100050 (CN); ZHANG, Jinlan, Beijing 100050 (CN); SHAO, Huihui, Beijing 100050 (CN); WANG, Yue, Beijing 100050 (CN); ZHANG, Yuanyuan, Beijing 100050 (CN)
(74) Representative: Kolster Oy Ab
(86) International application number: PCT/CN2023/090955
(87) International publication number: WO 2023/208068

(57) **Abstract**

Provided are an IMM-H014 sustained-release tablet for treating non-alcoholic fatty liver disease, hepatitis and drug-induced liver disease, and a preparation method therefor. The sustained-release tablet is prepared from an active pharmaceutical ingredient IMM-H014 or a pharmaceutically acceptable salt thereof, a framework material, a diluent, a glidant and a lubricant.

## Description

The present application is based on the application with CN application number 202210459865.9 and application date April 28, 2022, and claims its priority. The disclosure of the CN application is hereby introduced into the present application in its entirety.

### Technical Field

The present invention relates to a pharmaceutical preparation and a preparation method thereof, and in particular to an IMM-H014 sustained-release tablet for treating non-alcoholic fatty liver disease, hepatitis and drug-induced liver disease, and its preparation method and use, belonging to the field of pharmaceutical preparation.

### Background Art

Liver disease is a global disease and one of the common diseases that seriously endangers people's health. China is a major country for liver disease. At present, there are a large number of patients with liver damage and liver inflammation caused by various reasons, including viral hepatitis, drug-induced liver disease, alcoholic and non-alcoholic fatty liver disease, autoimmune liver disease, etc. Finding safe and effective drugs for the prevention and treatment of liver diseases has always been a hot spot for research and development by major research institutes and pharmaceutical companies around the world.

IMM-H014 is a biphenyl compound, an activator of nuclear factor NF-E2 (a core factor of cell defense)-related factor, which can simultaneously show effects of anti-inflammation and increasing insulin sensitivity, and can be used to treat non-alcoholic fatty liver disease, hepatitis and drug-induced liver disease (Wu Song, Sun Hua, Zhang Wenxuan, et al. A class of bicyclic alcohol derivatives and preparation and use thereof:, CN107488162A [P]).

The chemical name of IMM-H014 is: methyl 7,7'-dimethoxy-5'-(morpholinomethyl)-[4,4'-bibenzo[d][1,3]dioxole]-5-carboxylate.

### Chemical structure:

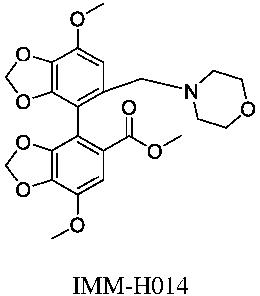

The commonly used IMM-H014 is a mesylate, and its solubility is pH-dependent, that is, as the pH increases, the solubility gradually decreases.

The Chinese invention patent CN107488162A discloses "a class of bicyclic alcohol derivatives and preparation and use thereof", in which the preparation and use of IMM-H014 mesylate are disclosed.

The international patent PCT201911190936.4 (application number) discloses "Use of levorotatory bicyclomorpholine and pharmaceutically acceptable salt thereof in drugs for prevention and/or treatment of liver disease", in which the preparation and use of levorotatory IMM-H014 mesylate are disclosed.

From the results of rat pharmacokinetic studies, it can be seen that the oral bioavailability of IMM-H014 in rats is 96.3%, indicating a high bioavailability. The compound shows rapid absorption by oral administration in rats and dogs, with a peak blood concentration of 0.5h, and a short elimination half-life after oral administration (the elimination half-life of oral administration in rats is about 1.8h, and the elimination half-life of oral administration in Beagle dogs is about 3.5h).

A drug with short half-life has short dosing interval and more dosing times, which will reduce the patient's compliance to a certain extent if the drug is used as a drug for the treatment of chronic diseases, thereby affecting the clinical application of the drug. In order to improve patient's compliance and reduce side effects, developing IMM-H014 into sustained-release tablets has important clinical value.

IMM-H014 sustained-release tablets are made by pressing active drug IMM-H014 or its pharmaceutically acceptable salt with one or more inert materials into tablets. According to the different properties of skeleton materials, skeleton-type sustained-release preparations can be divided into hydrophilic gel skeleton tablets, erodible skeleton tablets, and insoluble skeleton tablets. There are many dosage forms for the clinical application of skeleton preparations, and the most commonly used is oral dosage form.

### Contents of the Invention

One object of the present invention is to provide a prolonged-release tablet composition of biphenyl compound IMM-H014 or pharmaceutically acceptable salt thereof, and also provide a preparation method and use of the tablet.

The present invention adopts the following technical solution:
The present invention provides a sustained-release tablet of IMM-H014, wherein the active pharmaceutical ingredient is IMM-H014 or pharmaceutically acceptable salt thereof, and has a weight proportion of 5% to 25%, preferably 10% to 20%, in the sustained-release tablet.

The skeleton material of the sustained-release tablet of the present invention is selected from the group consisting of bioerodible skeleton, hydrophilic gel skeleton, and insoluble skeleton. Preferred hydrophilic gel skeleton materials include hydroxypropyl methylcellulose, hydroxypropyl cellulose, sodium carboxymethyl cellulose, sodium alginate, carbomer, chitosan, polyvinyl pyrrolidone, polyacrylic acid resins, beeswax, carnauba wax, stearyl alcohol, stearic acid, castor wax, glyceryl monostearate, triglyceride, and ethyl cellulose. More preferred is hydroxypropyl methylcellulose. The more preferred hydroxypropyl methylcellulose is hydroxypropyl methylcellulose with viscosity of K4M type. The weight proportion of the hydroxypropyl methylcellulose in the sustained-release tablet ranges from 10% to 80%, and the preferred range is 25% to 50%.

The diluent described in the present invention is one or a mixture of more than one of lactose, microcrystalline cellulose, starch, mannitol, sorbitol, sucrose, and calcium dihydrogen phosphate, preferably lactose. The dosage range of the diluent is 10% to 75%, and the preferred range is 35.5% to 65.5%.

The glidant described in the present invention is micro-powder silica gel. The dosage range of the glidant is 0.1% to 5%, preferably 0.5%.

The lubricant described in the present invention is one or a mixture of more than one of magnesium stearate, calcium stearate, talcum powder, and stearic acid. Preferably, it is magnesium stearate. The dosage range of the lubricant is 0.5% to 5%, preferably 1.5%.

A method for preparing an IMM-H014 sustained-release tablet, in which the IMM-H014 sustained-release tablet is prepared by powder direct compression method, comprises the following steps:
weighing IMM-H014 or pharmaceutically acceptable salt thereof, a skeleton material, and a diluent;
mixing evenly by equal amount incremental method;
adding a glidant and a lubricant, mixing evenly and pressing into tablets.

A method for preparing an IMM-H014 sustained-release tablet, in which the IMM-H014 sustained-release tablet is prepared by dry granulation tabletting method, comprises the following steps:
weighing IMM-H014 or pharmaceutically acceptable salt thereof, a skeleton material, and a diluent;
mixing evenly by equal amount incremental method;
performing dry granulating, granulating, then adding a glidant and a lubricant, mixing evenly, and pressing into tablets.

A method for preparing an IMM-H014 sustained-release tablet, in which the IMM-H014 sustained-release tablet is prepared by wet granulation tabletting method, comprises the following steps:
weighing IMM-H014 or pharmaceutically acceptable salt thereof, a skeleton material, and a diluent;
mixing evenly by equal amount incremental method;
adding an appropriate amount of adhesive solution for granulation, drying, and granulating, then adding a glidant and a lubricant, mixing evenly, and pressing into tablets. The adhesive is water, ethanol, ethanol aqueous solution, povidone solution, starch slurry, etc.

The *in vitro* release results of the IMM-H014 sustained-release tablets provided by the present invention show that the IMM-H014 sustained-release tablets have more obvious sustained-release effect than the IMM-H014 rapid-release tablets. As the amount of skeleton material increases, the release rate gradually slows down. And the preparation method can adopt powder direct compression, dry granulation tabletting, and wet granulation tabletting. The process is simple and controllable, with good reproducibility, low cost, and easy industrialization.

Beneficial technical effects: The IMM-H014 sustained-release tablets provided by the present invention and rapid-release tablets are subjected to *in vivo* pharmacokinetic determination in beagle dogs, and the results show that the IMM-H014 sustained-release tablets prepared by the present invention can significantly reduce the Cmax of drug and significantly prolong the MRT in comparison with the rapid-release tablets, indicating that there is an obvious sustained-release effect.

The following is only part examples of the present invention, and does not limit the present invention in any way. Any simple modification, change, and equivalent result change made to the examples according to the technical essence of the present invention are still within the protection scope of the technical solution of the present invention.

### Brief Description of the Drawings

Figure 1 shows the *in vitro* release curve of Test Example;
Figure 2 shows the drug-time curve in beagle dogs (Test Example);
Figure 3 shows the *in vitro* release curves of Test Example and Example 1;
Figure 4 shows the drug-time curves in beagle dogs (Test Example and Example 1);
Figure 5 shows the *in vitro* release curves of Test Example and Example 2;
Figure 6 shows the drug-time curves in beagle dogs (Test Example and Example 2);
Figure 7 shows the *in vitro* release curves of Test Example and Example 3;
Figure 8 shows the drug-time curves in beagle dogs (Test Example and Example 3);
Figure 9 shows the *in vitro* release curves of Test Example and Example 4;
Figure 10 shows the *in vitro* release curves of Test Example and Example 5;
Figure 11 shows the release curves of Test Example and Example 6;
Figure 12 shows the release curves of Test Example and Example 7.

### Specific Models for Carrying Out the Invention

The IMM-H014 sustained-release tablets of the present invention were subjected to *in vitro* release test and pharmacokinetic test.

### Release test:

The release was determined according to the first method of the general rules of the Chinese Pharmacopoeia.

Dissolution conditions: 750 ml of 0.1M hydrochloric acid solution was used as the dissolution medium, the rotation speed was 50 rpm, and after sampling at 0.5h, 1.0h, and 2.0h, 250 ml of 0.2M trisodium phosphate solution preheated to 37°C was immediately added, and the operation was continued according to the above method, and samples were taken at 3.0, 4.0, 5.0, 6.0, 8.0, 10.0, 12.0, 16.0, 20.0, and 24.0h.

Test solution: An appropriate amount of the dissolution solution was taken at each time point and filtered.

Reference solution: An appropriate amount of IMM-H014 reference substance was taken and added with 0.1M hydrochloric acid to prepare a 0.025 mg/ml solution.

Determination method: The test solution and the reference solution were taken, and measured for absorbance at a wavelength of 230nm according to the UV-visible spectrophotometry, and the cumulative release at each time point was calculated.

### Pharmacokinetics test:

Administration and sample collection: A two-cycle randomized crossover design was used, with a time interval of 7 days between two cycles. Ten beagles were randomly divided into two groups and orally administered with IMM-H014 rapid-release tablets (10 mg/kg) or IMM-H014 sustained-release tablets (10 mg/kg) after 12 hours of fasting. For the rapid-release tablets, 1 to 2 ml of forelimb venous blood was drawn before the administration (0 h) and at 0.08, 0.17, 0.25, 0.33, 0.5, 0.75, 1, 2, 3, 5, 8, 12, 24, 36, and 48 h after the administration; for the sustained-release tablets, 1 to 2 ml of forelimb venous blood was drawn before the administration (0 h) and at 0.25, 0.5, 1, 2, 3, 5, 8, 12, 24, 36, and 48 h after the administration. Heparin was used for anticoagulation, centrifugation was performed at 4000rpm for 10min at 4°C to separate plasma, which was stored at -20°C for later blood drug concentration analysis.

Chromatographic conditions: Chromatographic column was Agilent InfinityLab Poroshell 120 EC-C18 (2.1×50mm, 2.7µm); mobile phase A was 0.1% formic acid aqueous solution, mobile phase B was 0.1% formic acid in acetonitrile; flow rate was 0.4ml/min; injection volume was 1µL; column temperature was 35°C; internal standard was carbamazepine; gradient elution was adopted, elution program was: 0 to 2.50min, 20%B→40%B; 2.50 to 2.51min, 40%B-95%B; 2.51 to 3.5min, 95%B; 3.5 to 3.51min, 95%B→20%B; 3.51 to 4.5min, 20%B.

Mass spectrometry conditions: The LC interface used pneumatic assisted electrospray ionization source (ESI) conditions; detection mode: positive ion; drying gas: 150°C, nitrogen, 11 L/min; atomization gas: nitrogen, 25psi; sheath gas: 300°C, 11L/min; capillary voltage: 3000V; scanning mode: multiple reaction monitoring (MRM); detection ion pair was m/z: 460.1→341.

Preparation of plasma standard curve and quality control samples: An appropriate amount of H014 working solution (100µg/ml) was taken and diluted gradiently with 20% acetonitrile aqueous solution to prepare a series of standard solutions with concentrations of 0.005, 0.02, 0.05, 0.5, 1, 5, 10, 20, 40 and 80 µg/ml, respectively. The series of standard solutions were taken at 10µl, respectively, and added with 90µl of blank plasma, vortexed for 30s, to prepare a series of plasma standard solutions with H014 concentrations of 0.5, 2, 5, 50, 100, 500, 1000, 2000, 4000 and 8000 ng/ml, respectively; then added with 300µl of internal standard working solution (500ng/ml), respectively, vortexed for 2 min, centrifuged at 13000rpm and 4°C for 5min, and supernatants were taken as standard curve solutions, and placed into injection bottles for injection and determination. With the reciprocal of H014 concentration as the weight, the peak area ratio of H014 to internal standard was used to perform a linear regression on the concentration ratio of H014 to internal standard. The standard curve equation was y=0.9983x-0.0101. The linear range of the drug in beagle dog plasma was 0.5 to 8000 µg/ml, and the correlation coefficient r2 was all >0.99. The concentrations of quality control samples (biological sample concentrations) were 5, 500 and 4000 ng/ml, respectively.

Preparation of samples to be tested: 100µl plasma sample was taken and added with 300µl of internal standard working solution in a 1.5ml centrifuge tube, vortexed for 2min, centrifuged at 13000 rpm and 4°C for 5min, 50 µl of supernatant was taken and placed in a sample injection vial, added with 950µl of diluent and mixed well for later testing.

### Test Example: Preparation of IMM-H014 rapid-release tablets

**Table 1: Prescription of Test Example**

| Raw material | % (w/w) | Charge for preparing 1000 tablets (g) |
|---|---|---|
| IMM-H014 (mesylate) | 12.5 | 25 |
| Microcrystalline cellulose (102) | 60 | 120 |
| Pregelatinized starch | 25.5 | 51 |
| Micro-powder silica gel | 0.5 | 1 |
| Magnesium stearate | 1.5 | 3 |

IMM-H014 was mixed with microcrystalline cellulose and pregelatinized starch by equal amount incremental method. Micro-powder silica gel and magnesium stearate were added and mixed for 10 minutes. Tablets were pressed in an 8mm die, with a tablet weight of 200mg and a hardness of 6 to 9 kg.

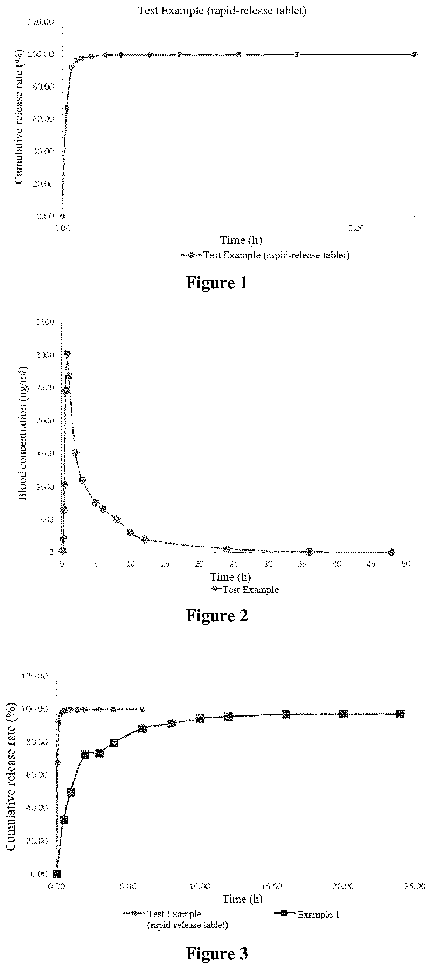

The determination of *in vitro* release rate, the determination of blood drug concentration in beagle dogs, and the calculation of pharmacokinetic parameters were performed according to the above experimental methods. The results were shown in Table 2, Table 3, Figure 1, and Figure 2.

**Table 2: Release rate of Test Example**

| Time (h) | Release rate (%) | Time (h) | Release rate (%) |
|---|---|---|---|
| 0.00 | 0.0 | 1.0 | 99.6 |
| 0.08 | 67.3 | 1.5 | 99.6 |
| 0.17 | 92.0 | 2.0 | 99.7 |
| 0.25 | 96.0 | 3.0 | 99.7 |
| 0.33 | 97.4 | 4.0 | 99.8 |
| 0.50 | 98.6 | 6.0 | 99.8 |
| 0.75 | 99.5 | | |

**Table 3: Pharmacokinetic results of Test Example**

| Pharmacokinetic parameter | Unit | Experimental result |
|---|---|---|
| AUC(0-t) | ug/L*h | 12925.495 |
| AUC(0-∞) | ug/L*h | 13010.006 |
| MRT(0-t) | h | 5.052 |
| MRT(O-oo) | h | 5.448 |
| t1/2 (calculated) | h | 3.775 |
| Tmax | h | 0.5 |
| Cmax | ug/L | 4776.103 |

Example 1: Preparation of IMM-H014 sustained-release tablets by powder direct compression method

**Table 4: Prescription of Example 1**

| Raw material | % (w/w) | Charge for preparing 1000 tablets (g) |
|---|---|---|
| IMM-H014 (mesylate) | 12.5 | 25 |
| Hydroxypropyl methylcellulose (K4M) | 15 | 30 |
| Lactose | 70.5 | 141 |
| Micro-powder silica gel | 0.5 | 1 |
| Magnesium stearate | 1.5 | 3 |

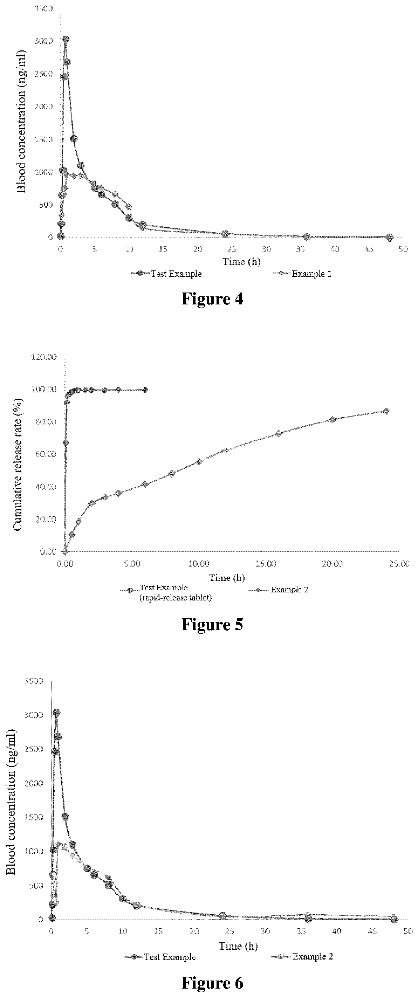

IMM-H014 together with hydroxypropyl methylcellulose and lactose were sieved, and mixed well by equal amount incremental method. Micro-powder silica gel and magnesium stearate were added and mixed for 10 minutes. Tablets were pressed in an 8mm die, with a tablet weight of 200mg and a hardness of 10 to 13 kg. The determination of *in vitro* release rate, the determination of blood drug concentration in beagle dogs, and the calculation of pharmacokinetic parameters were performed according to the above experimental methods. The results were shown in Table 5, Table 6, Figure 3, and Figure 4.

**Table 5: Release rate of Example 1**

| Time (h) | Release rate (%) | Time (h) | Release rate (%) |
|---|---|---|---|
| 0.0 | 0.0 | 8 | 91.3 |
| 0.5 | 32.8 | 10 | 94.3 |
| 1 | 49.6 | 12 | 95.5 |
| 2 | 72.5 | 16 | 96.8 |
| 3 | 73.3 | 20 | 96.9 |
| 4 | 79.6 | 24 | 97.1 |
| 6 | 88.2 | | |

**Table 6: Pharmacokinetic results of Example 1**

| Pharmacokinetic parameter | Unit | Example 1 |
|---|---|---|
| AUC(0-t) | ug/L*h | 11459.455 |
| AUC(0-∞) | ug/L*h | 11495.26 |
| MRT(0-t) | h | 8.372 |
| MRT(O-oo) | h | 8.727 |
| t1/2 (calculated) | h | 6.048 |
| Tmax | h | 3.1 |
| Cmax | ug/L | 1276.848 |

Example 2: Preparation of IMM-H014 sustained-release tablets by powder direct compression method

**Table 7: Prescription of Example 2**

| Raw material | % (w/w) | Charge for preparing 1000 tablets (g) |
|---|---|---|
| IMM-H014 (mesylate) | 12.5 | 25 |
| Hydroxypropyl methylcellulose (K4M) | 32.5 | 65 |
| Lactose | 53 | 106 |
| Micro-powder silica gel | 0.5 | 1 |
| Magnesium stearate | 1.5 | 3 |

IMM-H014 was mixed well with hydroxypropyl methylcellulose and lactose by equal amount incremental method. Micro-powder silica gel and magnesium stearate were added and mixed for 10 minutes. Tablets were pressed in an 8mm die, with a tablet weight of 200 mg and a hardness of 10 to 13 kg. The determination of *in vitro* release rate, the determination of blood drug concentration in beagle dogs, and the calculation of pharmacokinetic parameters were performed according to the above experimental methods. The results were shown in Table 8, Table 9, Figure 5, and Figure 6.

**Table 8: Release rate of Example 2**

| Time (h) | Release rate (%) | Time (h) | Release rate (%) |
|---|---|---|---|
| 0.0 | 0.0 | 8 | 48.3 |
| 0.5 | 10.8 | 10 | 55.6 |
| 1 | 18.5 | 12 | 62.4 |
| 2 | 30.1 | 16 | 73.0 |
| 3 | 33.5 | 20 | 81.4 |
| 4 | 36.0 | 24 | 87.0 |
| 6 | 41.5 | | |

**Table 9: Pharmacokinetic results of Example 2**

| Pharmacokinetic parameter | Unit | Example 2 |
|---|---|---|
| AUC(0-t) | ug/L*h | 10894.311 |
| AUC(0-∞) | ug/L*h | 11071.683 |
| MRT(0-t) | H | 9.315 |
| MRT(0-∞) | H | 11.064 |
| t1/2 (calculated) | H | 7.667 |
| Tmax | H | 2.5 |
| Cmax | ug/L | 1364.039 |

Example 3: Preparation of IMM-H014 sustained-release tablets by powder direct compression method

**Table 10: Prescription of Example 3**

| Raw material | % (w/w) | Charge for preparing 1000 tablets (g) |
|---|---|---|
| IMM-H014 (mesylate) | 12.5 | 25 |
| Hydroxypropyl methylcellulose (K4M) | 45 | 90 |
| Lactose | 40.5 | 81 |
| Micro-powder silica gel | 0.5 | 1 |
| Magnesium stearate | 1.5 | 3 |

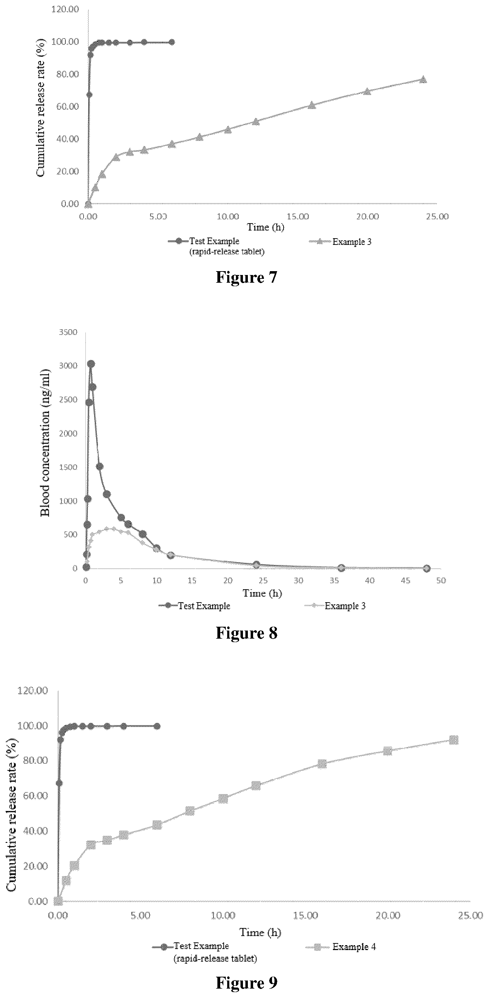

IMM-H014 was mixed well with hydroxypropyl methylcellulose and lactose by equal amount incremental method. Micro-powder silica gel and magnesium stearate were added and mixed for 10 minutes. Tablets were pressed in an 8mm die, with a tablet weight of 200 mg and a hardness of 10 to 13 kg. The determination of *in vitro* release rate, the determination of blood drug concentration in beagle dogs, and the calculation of pharmacokinetic parameters were performed according to the above experimental methods. The results were shown in Table 11, Table 12, Figure 7, and Figure 8.

**Table 11: Release rate of Example 3**

| Time (h) | Release rate (%) | Time (h) | Release rate (%) |
|---|---|---|---|
| 0.0 | 0.0 | 8 | 41.4 |
| 0.5 | 10.2 | 10 | 46.1 |
| 1 | 18.6 | 12 | 51.1 |
| 2 | 29.1 | 16 | 61.0 |
| 3 | 32.4 | 20 | 69.8 |
| 4 | 33.4 | 24 | 77.2 |
| 6 | 37.1 | | |

**Table 12: Pharmacokinetic results of Example 3**

| Pharmacokinetic parameter | Unit | Example 3 |
|---|---|---|
| AUC(0-t) | ug/L*h | 7159.013 |
| AUC(0-∞) | ug/L*h | 7191.927 |
| MRT(0-t) | H | 8.595 |
| MRT(O-oo) | H | 8.812 |
| t1/2 (calculated) | H | 6.107 |
| Tmax | H | 3.125 |
| Cmax | ug/L | 739.448 |

Example 4: Preparation of IMM-H014 sustained-release tablets by powder direct compression method

**Table 13: Prescription of Example 4**

| Raw material | % (w/w) | Charge for preparing 1000 tablets (g) |
|---|---|---|
| IMM-H014 (mesylate) | 12.5 | 25 |
| Ethyl cellulose (E45) | 40 | 80 |
| Lactose | 45.5 | 91 |
| Micro-powder silica gel | 0.5 | 1 |
| Magnesium stearate | 1.5 | 3 |

IMM-H014 was mixed well with ethyl cellulose and lactose by equal amount incremental method. Micro-powder silica gel and magnesium stearate were added and mixed for 10 minutes. Tablets were pressed in an 8mm die, with a tablet weight of 200 mg and a hardness of 10 to 13 kg. The determination of *in vitro* release rate was performed according to the above experimental method. The results were shown in Table 14, and Figure 9.

**Table 14: Release rate of Example 4**

| Time (h) | Release rate (%) | Time (h) | Release rate (%) |
|---|---|---|---|
| 0.0 | 0.0 | 8 | 51.4 |
| 0.5 | 11.8 | 10 | 58.6 |
| 1 | 20.5 | 12 | 65.8 |
| 2 | 32.1 | 16 | 78.2 |
| 3 | 34.8 | 20 | 85.7 |
| 4 | 37.9 | 24 | 92.1 |
| 6 | 43.6 | | |

Example 5: Preparation of IMM-H014 sustained-release tablets by dry granulation tabletting method

**Table 15: Prescription of Example 5**

| Raw material | % (w/w) | Charge for preparing 1000 tablets (g) |
|---|---|---|
| IMM-H014 (mesylate) | 12.5 | 25 |
| Stearic acid | 30 | 60 |
| Lactose | 55.5 | 111 |
| Micro-powder silica gel | 0.5 | 1 |
| Magnesium stearate | 1.5 | 3 |

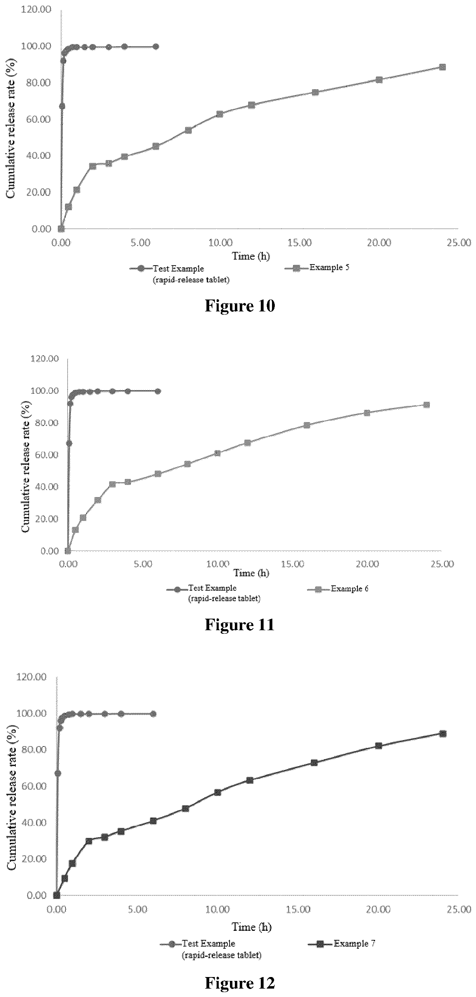

IMM-H014 was mixed well with stearic acid and lactose by equal amount incremental method. After dry granulation, and granulating, micro-powder silica gel and magnesium stearate were added and mixed for 10 minutes. Tablets were pressed in an 8 mm die, with a tablet weight of 200 mg and a hardness of 10 to 13 kg. The determination of *in vitro* release rate was performed according to the above experimental method. The results were shown in Table 16 and Figure 10.

**Table 16: Release rate of Example 5**

| Time (h) | Release rate (%) | Time (h) | Release rate (%) |
|---|---|---|---|
| 0.0 | 0.0 | 8 | 54.2 |
| 0.5 | 12.1 | 10 | 62.7 |
| 1 | 21.6 | 12 | 67.8 |
| 2 | 34.3 | 16 | 74.8 |
| 3 | 35.9 | 20 | 81.6 |
| 4 | 39.5 | 24 | 88.6 |
| 6 | 45.1 | | |

Example 6: Preparation of IMM-H014 sustained-release tablets by dry granulation tabletting method

**Table 17: Prescription of Example 6**

| Raw material | % (w/w) | Charge for preparing 1000 tablets (g) |
|---|---|---|
| IMM-H014 (mesylate) | 12.5 | 25 |
| Hydroxypropyl methylcellulose (K4M) | 32.5 | 65 |
| Lactose | 53 | 106 |
| Micro-powder silica gel | 0.5 | 1 |
| Magnesium stearate | 1.5 | 3 |

IMM-H014 was mixed well with hydroxypropyl methylcellulose and lactose by equal amount incremental method. After dry granulation, and granulating, micro-powder silica gel and magnesium stearate were added and mixed for 10 minutes. Tablets were pressed in an 8 mm die, with a tablet weight of 200 mg and a hardness of 10 to 13 kg. The determination of *in vitro* release rate was performed according to the above experimental method. The results were shown in Table 18 and Figure 11.

**Table 18: Release rate of Example 6**

| Time (h) | Release rate (%) | Time (h) | Release rate (%) |
|---|---|---|---|
| 0.0 | 0.0 | 8 | 54.5 |
| 0.5 | 13.2 | 10 | 61.1 |
| 1 | 20.7 | 12 | 67.6 |
| 2 | 32.0 | 16 | 78.6 |
| 3 | 41.9 | 20 | 86.4 |
| 4 | 43.1 | 24 | 91.4 |
| 6 | 48.1 | | |

Example 7: Preparation of IMM-H014 sustained-release tablets by wet granulation tabletting method

**Table 19: Prescription of Example 7**

| Raw material | % (w/w) | Charge for preparing 1000 tablets (g) |
|---|---|---|
| IMM-H014 (mesylate) | 12.5 | 25 |
| Hydroxypropyl methylcellulose (K4M) | 32.5 | 65 |
| Lactose | 53 | 106 |
| Micro-powder silica gel | 0.5 | 1 |
| Magnesium stearate | 1.5 | 3 |

IMM-H014 was mixed well with hydroxypropyl methylcellulose and lactose by equal amount incremental method. Granulation was performed with ethanol/water (80%) as wetting agent, and the granules were dried at 50°C, passed through 18 mesh sieve, added with micro-powder silica gel and magnesium stearate, and mixed for 10 min. Tablets were pressed in an 8 mm die, with a tablet weigh of 200mg, and a hardness of 10 to 13 kg. The determination of *in vitro* release rate was performed according to the above experimental method. The results were shown in Table 20 and Figure 12.

**Table 20: Release of Example 7**

| Time (h) | Release rate (%) | Time (h) | Release rate (%) |
|---|---|---|---|
| 0.0 | 0.0 | 8 | 47.8 |
| 0.5 | 9.4 | 10 | 56.7 |
| 1 | 17.8 | 12 | 63.2 |
| 2 | 29.7 | 16 | 72.8 |
| 3 | 32.2 | 20 | 82.0 |
| 4 | 35.4 | 24 | 89.0 |
| 6 | 41.0 | | |

## Claims

1. A skeleton sustained-release tablet of drug IMM-H014, **characterized in that** the sustained-release tablet has weight percentage composition as follows: 5% to 25% of IMM-H014 as shown in Formula 1 or pharmaceutically acceptable salt thereof as active ingredient, 10% to 80% of a skeleton material, 10% to 75% of a diluent, 0.1% to 5% of a glidant, and 0.5% to 5% of a lubricant,

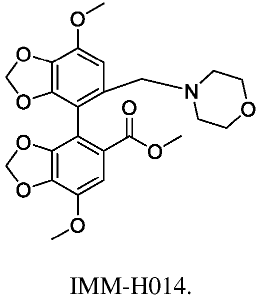

2. The skeleton sustained-release tablet of drug IMM-H014 according to claim 1, **characterized in that** the skeleton material is one or more selected from the group consisting of hydroxypropyl methylcellulose, hydroxypropyl cellulose, sodium alginate, chitosan, carbomer, polyvinyl pyrrolidone, acrylic resin, beeswax, carnauba wax, stearyl alcohol, stearic acid, castor wax, glyceryl monostearate, triglyceride, and ethyl cellulose.

3. The skeleton sustained-release tablet of drug IMM-H014 according to claim 2, **characterized in that** the hydroxypropyl methylcellulose comprises those with viscosity type of K4M, K15M, or K100M, and the hydroxypropyl cellulose comprises those with viscosity type of HPC-M or HPC-H.

4. The skeleton sustained-release tablet of drug IMM-H014 according to claim 1, **characterized in that** the diluent is one or a mixture of more than one selected from the group consisting of lactose, microcrystalline cellulose, starch, mannitol, sorbitol, sucrose, and calcium dihydrogen phosphate; the glidant is micro-powder silica gel; the lubricant is one or a mixture of more than one selected from the group consisting of magnesium stearate, calcium stearate, talc, and stearic acid.

5. A method for preparing the skeleton sustained-release tablet of drug IMM-H014 according to any one of claims 1 to 4, **characterized in that** the skeleton sustained-release tablet of drug IMM-H014 is prepared by powder direct compression method, comprising the following steps:
weighing IMM-H014 or pharmaceutically acceptable salt thereof, a sustained-release skeleton material, and a diluent;
mixing evenly by equal amount incremental method;
adding a glidant and a lubricant, mixing evenly and pressing into tablets.

6. A method for preparing the skeleton sustained-release tablet of drug IMM-H014 according to any one of claims 1 to 4, **characterized in that** the skeleton sustained-release tablet of drug IMM-H014 is prepared by dry granulation tabletting method, comprising the following steps:
weighing IMM-H014 or pharmaceutically acceptable salt thereof, a sustained-release skeleton material, and a diluent;
mixing evenly by equal amount incremental method;
performing dry granulation, granulating, then adding a glidant and a lubricant, mixing evenly, and pressing into tablets.

7. A method for preparing the skeleton sustained-release tablet of drug IMM-H014 according to any one of claims 1 to 4, **characterized in that** the sustained-release tablet of IMM-H014 is prepared by wet granulation tabletting method, comprising the following steps:
weighing IMM-H014 or pharmaceutically acceptable salt thereof, a sustained-release skeleton material, and a diluent;
mixing evenly by equal amount incremental method;
adding an appropriate amount of binder solution to perform wet granulation, drying, and granulating, then adding a glidant and a lubricant, mixing evenly, and pressing into tablets; the binder is selected from the group consisting of water, ethanol, ethanol aqueous solution, povidone solution, and starch slurry.

8. Use of the skeleton sustained-release tablet of drug IMM-H014 according to any one of claims 1 to 4 in the manufacture of a medicament for preventing and/or treating a liver disease.

9. The use according to claim 8, **characterized in that** the liver disease is selected from the group consisting of hepatitis A, hepatitis B, hepatitis C, drug-induced liver disease, alcoholic liver disease, non-alcoholic liver disease, autoimmune liver disease, liver fibrosis of liver disease progression, cirrhosis or liver failure.
